# FASCICULE DE BREVET EUROPEEN

(11) **EP 3 871 658 B1**
(45) Date de publication et mention de la délivrance du brevet: **15.01.2025**
(21) Numéro de dépôt: 21159318.1
(22) Date de dépôt: 25.02.2021
(51) Int. Cl.: A61K 8/9789, A61Q 5/00, A61Q 5/10

(54) **PRINCIPE ACTIF COMPRENANT UN EXTRAIT D'AVOINE NOIRE ET UN EXTRAIT DE BUGRANE EPINEUSE ET UTILISATIONS COSMETIQUES NOTAMMENT ANTI-GRISONNEMENT**
WIRKSTOFF, DER EIN EXTRAKT AUS SCHWARZEM HAFER UND EIN EXTRAKT AUS DORNIGEM HAUHECHEL ENTHÄLT, UND KOSMETISCHE ANWENDUNGEN INSBESONDERE GEGEN HAARERGRAUEN
ACTIVE INGREDIENT COMPRISING AN EXTRACT OF BLACK OATS AND AN EXTRACT OF SPINY RESTHARROW AND COSMETIC USES, IN PARTICULAR, HAIR ANTI-GREYING

(30) Priorité: 25.02.2020 FR 2001842
(43) Date de publication de la demande: 01.09.2021
(73) Titulaire: Societe Industrielle Limousine d'Application Biologique, 19130 Objat (FR)
(72) Inventeur: PAUFIQUE, Jean, 19130 OBJAT (FR)
(74) Mandataire: Aquinov

(56) Documents cités:
- CN-A- 108 618 133
- JP-A- 2002 080 382
- US-A1- 2019 336 884

## Description

### Domaine de l'invention

L'invention concerne un principe actif cosmétique spécifique comprenant au moins un extrait *d'Avena strigosa* combiné à au moins un extrait *d'Ononis spinosa,* utile en cosmétique en particulier pour prévenir et/ou lutter contre le grisonnement des cheveux. L'invention concerne également les compositions cosmétiques l'incluant, un procédé d'obtention d'un tel principe actif ainsi que ses utilisations cosmétiques notamment sur les cheveux.

### Etat de l'art

Des recherches récentes ont démontré que l'aspect des cheveux est un attribut majeur dans l'interprétation de l'âge, de la santé et de l'attractivité d'un sujet. La chevelure est ainsi un réel outil de communication et de séduction et possède un pouvoir important dans la construction de l'identité et de l'image qu'une personne souhaite renvoyer.

Dans ce contexte, l'apparition de cheveux gris, appelée canitie ou grisonnement des cheveux, bien qu'étant un phénomène normal intervenant avec le vieillissement, est malheureusement souvent mal vécue. Une étude a ainsi démontré que les femmes aux cheveux poivre et sel sont vieillies de 6 ans par rapport à leur âge réel, tandis que les hommes sont perçus plus âgés de 3 ans. Dans un environnement social où les impératifs de jeunisme restent prégnants, les cheveux gris transmettent une image biaisée de vieillissement et sont parfois perçus comme du laisser-aller, d'où le souhait légitime de les dissimuler.

Toutefois, actuellement, pour masquer ses cheveux gris il est nécessaire d'utiliser des colorations capillaires. Ces dernières demeurant contraignantes, il existe un besoin important pour un produit capable de faire disparaître les cheveux gris et/ou de prévenir leur apparition.

JP2002080382A (KAO CORP.) propose l'utilisation de plusieurs extraits végétaux dont l'extrait d'Ononis spinosa comme principe actif pour traiter le grisonnement capillaire.

### Résumé de l'invention

L'objectif de l'invention est de proposer un principe actif capable de diminuer le nombre de cheveux gris notamment en favorisant la repigmentation de la fibre capillaire et en améliorant la qualité structurale des cheveux.

A cet effet, l'invention a pour objet un principe actif cosmétique comprenant au moins un extrait *d'Avena strigosa* et également au moins un extrait *d'Ononis spinosa. Avena strigosa* ou l'avoine noire, est une céréale d'originaire de l'Ouest méditerranéen. Il s'agit d'une céréale rustique, cultivée en Europe depuis plusieurs millénaires notamment pour l'intérêt nutritionnel de ses graines riches en protéines et lipides. La culture de l'avoine noire en alternance d'autres plantes est appréciée pour ses pouvoirs allélopathiques qui inhibent le développement de végétaux non désirés mais aussi pour son puissant système racinaire améliorant la structure du sol. Une fois l'avoine noire récoltée, une partie de la biomasse mise en jachère procure un apport de matières organiques au sol (engrais vert) et lutte contre le développement de phytoparasites.

*Ononis spinosa* ou bugrane épineuse, est un arbuste originaire des prairies et pâturages semi- arides d'Europe, d'Asie Occidentale et du Nord de l'Afrique. Cette plante de petite taille est reconnaissable à ses fleurs papilionacées dont la corolle rose poudrée évoque la forme éponyme du papillon. Il s'agit d'une plante, dotée de racines solides. Les racines de la bugrane sont connues pour leurs propriétés diurétique et anti-lithiotique. En médecine traditionnelle, elles sont utilisées pour stimuler la diurèse, ainsi que pour leurs propriétés analgésiques et anti-bactériennes. La valorisation de la bugrane relève également d'un savoir-faire artisanal car la plante était employée en Iran pour la production de couleurs.

Or, de façon surprenante, les extraits *d'Avena strigosa* et *d'Ononis spinosa,* lorsqu'ils sont appliqués sur une chevelure, permettent d'améliorer efficacement la lutte contre le grisonnement des cheveux, tant en prévention qu'en traitement.

L'invention a par conséquent pour objet un principe actif cosmétique particulier à savoir un principe actif cosmétique comprenant au moins un extrait *d'Avena strigosa* et au moins un extrait *d'Ononis spinosa,* ainsi que son utilisation cosmétique en application sur les cheveux, pour améliorer un effet anti-grisonnement.

L'invention a également pour objet un procédé d'obtention d'un tel principe actif ainsi que les compositions cosmétiques adaptées à une application capillaire l'incluant. Avantageusement, l'utilisation combinée *d'Avena strigosa* et *d'Ononis spinosa* permet de lutter efficacement contre le stress oxydatif du cheveu gris pour un effet anti-grisonnement important, visible et durable.

D'autres caractéristiques et avantages ressortiront de la description détaillée de l'invention qui va suivre réalisée en regard notamment des exemples, résultats d'essais et figures.

### Figures

[Figure 1] La figure 1 représente les résultats de l'évolution de la mélanine en fonction de la pigmentation du cheveu dans la modélisation de la signature moléculaire du grisonnement des cheveux.
[Figure 2] La figure 2 représente les résultats de l'analyse de l'hydrogène peroxydé dans la modélisation de la signature moléculaire du grisonnement des cheveux.
[Figure 3] La figure 3 représente les résultats de l'analyse des acides aminés oxydés dans la modélisation de la signature moléculaire du grisonnement des cheveux.
[Figure 4] La figure 4 représente les résultats de l'analyse de la structure secondaire des protéines (hélices α / feuillets β) dans la modélisation de la signature moléculaire du grisonnement des cheveux.

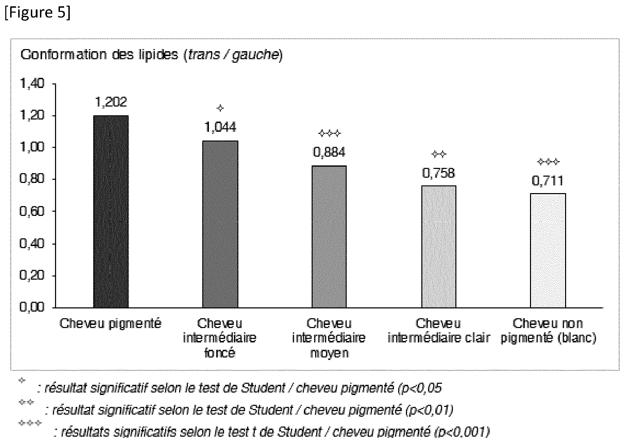

[Figure 5] La figure 5 représente les résultats de l'analyse de la conformation des lipides dans la modélisation de la signature moléculaire du grisonnement des cheveux.

### Définitions

Par « actif cosmétique » ou « principe actif cosmétique » au sens de l'invention, on entend au moins une molécule, préférentiellement un ensemble de molécules présentant un effet cosmétique sur la peau ou les cheveux.

Par « extrait » d'une matière première X, au sens de la présente invention, on entend toute molécule ou mélange d'au moins deux molécules obtenu(e) à partir d'une matière première X, quel que soit le procédé d'extraction de ladite ou desdites molécule(s). Il peut s'agir par exemple d'un extrait obtenu par extraction aqueuse et/ou hydroalcoolique et/ou hydroglycolique et/ou hydrolyse(s), etc.

Par « hydrolysat » d'une matière première X au sens de l'invention, on entend tout extrait obtenu à partir d'une matière première X, par un procédé comprenant au moins une étape d'hydrolyse enzymatique ou chimique.

Par « hydrolysat enzymatique » d'une matière première X au sens de l'invention, on entend tout extrait obtenu à partir d'une matière première X par un procédé comprenant au moins une étape d'hydrolyse enzymatique.

Par « extrait hydroglycolique » d'une matière première X au sens de l'invention, on entend tout extrait obtenu à partir d'une matière première X par un procédé comprenant au moins une étape d'extraction à l'aide d'au moins un mélange d'au moins deux solvants (eau et un solvant glycolique, comme par exemple le butylène glycol).

### Description détaillée de l'invention

L'invention vise donc un principe actif spécifique, particulièrement utile en cosmétique. Ledit principe actif est un principe actif cosmétique comprenant au moins un extrait *d'Avena strigosa* et au moins un extrait *d'Ononis spinosa.*

Préférentiellement le principe actif cosmétique selon l'invention comprend au moins un hydrolysat *d'Avena strigosa.* Il peut s'agir d'un hydrolysat enzymatique *d'Avena strigosa,* notamment un hydrolysat enzymatique obtenu à l'aide d'une ou plusieurs protéases. Préférentiellement le principe actif selon l'invention comprend au moins un extrait *d'Avena strigosa* obtenu à partir de graines *d'Avena strigosa.*

Préférentiellement le principe actif selon l'invention comprend au moins un extrait hydroglycolique *d'Ononis spinosa.*

Préférentiellement le principe actif selon l'invention comprend au moins un extrait *d'Ononis spinosa* obtenu à partir de racines *d'Ononis spinosa.*

Selon un mode de réalisation préféré, le principe actif cosmétique selon l'invention comprend au moins un extrait de graines *d'Avena strigosa* et au moins un extrait de racines *d'Ononis spinosa.*

De façon préférée, le principe actif cosmétique selon l'invention, comprend au moins une fraction peptidique. Préférentiellement, la fraction peptidique du principe actif selon l'invention comprend au moins des peptides de masses molaires inférieures à 2000 Da. Selon un mode de réalisation particulièrement adapté, les peptides de masses molaires inférieures à 2000 Da représentent préférentiellement entre 21% et 53% en poids de matière sèche du principe actif selon l'invention, notamment environ 30% en poids de matière sèche du principe actif selon l'invention (soit 30% soit 30% plus ou moins 5%).

Le dosage des peptides dans le principe actif selon l'invention peut être réalisé notamment par la méthode de KJELDHAL (référence : Official method of analysis of the A.O.C., 12th ed. W Horwitz, E.D., New-York, 15-60, 1975). La teneur en protéines est exprimée en pourcentage par rapport à la teneur en matières sèches. De plus, pour déterminer la taille des composés peptidiques présents dans le principe actif selon l'invention, il est possible de réaliser une chromatographie de type F.P.L.C.

Selon un autre aspect, le principe actif selon l'invention peut comprendre au moins des composés phénoliques, préférentiellement au moins des acides phénoliques et des flavonoïdes.. Dans le cas où le principe actif selon l'invention comprend des composés polyphénoliques, ils peuvent représenter préférentiellement entre 11% et 29% en poids de matière sèche du principe actif selon l'invention, notamment environ 22% en poids de matière sèche du principe actif (soit 22% soit 22% plus ou moins 5%).

La teneur en composés phénoliques peut être déterminée par dosage colorimétrique. Les composés phénoliques forment en présence de ferricyanure de potassium et de chlorure de fer des complexes colorés qui peuvent être dosés par spectrophotométrie à 715 nm. L'intensité de cette coloration est proportionnelle à la quantité de composés phénoliques présents dans l'échantillon. Des solutions de calibration sont préparées à partir d'un standard d'hespéridine de 40 à 120 mg/L. Une courbe de calibration des densités optiques des solutions de calibration en fonction de leur concentration est construite. Les échantillons sont préalablement dilués avec de l'eau distillée pour que la teneur en polyphénols corresponde à la gamme d'étalonnage. La quantité de polyphénols des échantillons est déterminée grâce à la courbe de calibration.

Les composés phénoliques du principe actif selon l'invention peuvent être caractérisés par toute méthode adaptée en particulier par chromatographie liquide couplée à une détection UV.

Le principe actif selon l'invention peut contenir également des carbohydrates et/ou des minéraux.

Ainsi le principe actif selon l'invention peut comprendre :
- des peptides, et/ou
- des composés phénoliques et/ou
- des carbohydrates, et/ou
- des minéraux.

Préférentiellement, le principe actif selon l'invention comprend au moins :
- des peptides (biopeptides) et des composés phénoliques, et
- éventuellement des carbohydrates, et/ou des minéraux.

Le principe actif selon l'invention peut se présenter sous forme solide ou sous forme liquide. Lorsqu'il se présente sous forme liquide, le principe actif selon l'invention est préférentiellement exclusivement constitué par un ou plusieurs extrait(s) liquide(s) *d'Avena strigosa* et un ou plusieurs extrait(s) liquide(s) *d'Ononis spinosa.*

Préférentiellement le ratio extrait *d'Avena strigosa* / extrait *d'Ononis spinosa* est compris entre 45/55 et 55/45 en volume.

Le principe actif selon l'invention sous forme liquide se présente préférentiellement sous forme d'un liquide limpide, avec une faible odeur et une couleur ambrée. Il peut toutefois être plus coloré et/ou être décoloré par tout procédé connu de l'homme du métier. Préférentiellement, le principe actif selon l'invention sous forme liquide a une teneur en matières sèches de : 10 g/L à 50g/L, encore plus préférentiellement de 20 g/L à 32g/L. Lorsqu'il se présente sous forme solide, notamment sous forme de poudre, le principe actif selon l'invention est constitué par :
- un support tel par exemple que les maltodextrines, et
- au moins un extrait *d'Avena strigosa* et au moins un extrait *d'Ononis spinosa.*

Dans ce cas, préférentiellement, le ou les extraits représente(nt) au moins 10% en poids du principe actif et le support au plus 90%.

Le principe actif selon l'invention peut aussi être présenté sous forme d'un film sec. Dans ce cas, la combinaison extrait *d'Avena strigosa* et extrait *d'Ononis spinosa* représente préférentiellement au moins 0.1% en poids du film sec.

L'extrait *d'Avena strigosa* présent dans le principe actif selon l'invention peut être obtenu par tout moyen. Préférentiellement le principe actif selon l'invention comprend au moins un extrait obtenu par la mise en œuvre des étapes suivantes :
- solubilisation *d'Avena strigosa,* préférentiellement de graines *d'Avena strigosa,* dans l'eau à raison d'au moins 100 g/L
- hydrolyse(s) enzymatique(s), préférentiellement à l'aide de protéases, en suivant les instructions du fournisseur,
- séparation des phases soluble et insoluble par tout moyen permettant de séparer une phase insoluble de la phase soluble, par exemple par centrifugation, filtration ou décantation
- préférentiellement inactivation des activités enzymatiques par tout moyen permettant l'inactivation des activités enzymatiques, par exemple par traitement thermique
- préférentiellement filtration(s) successive(s) permettant de sélectionner le filtrat, par exemple par filtre presse, ultrafiltration, nanofiltration,
- éventuellement désodorisation ou décoloration par ajout d'un adjuvant de procédé permettant la désodorisation ou la décoloration.

L'extrait *d'Ononis spinosa* présent dans le principe actif selon l'invention peut être obtenu par tout moyen. Préférentiellement le principe actif selon l'invention comprend au moins un extrait *d'Ononis spinosa* obtenu par la mise en œuvre des étapes suivantes :
- solubilisation à au moins 100g/L dans une solution hydroglycolique, préférentiellement une solution eau/Butylène Glycol, de façon préférée 50/50 (v/v)
- séparation des phases soluble et insoluble par tout moyen permettant de séparer une phase insoluble de la phase soluble, par exemple décantation, filtration ou centrifugation
- préférentiellement filtration(s) successive(s) permettant de sélectionner le filtrat, par exemple par filtre presse, ultrafiltration, nanofiltration.

Préférentiellement, les deux extraits liquides sont mélangés ensemble pour obtenir le principe actif selon l'invention sous forme liquide. Préférentiellement cette étape de mélange est suivie d'une ou plusieurs étapes de filtration.

Un exemple de procédé d'obtention d'un principe actif selon l'invention comprenant au moins un extrait *d'Avena strigosa* et au moins un extrait *d'Ononis spinosa,* est un procédé comprenant les étapes suivantes :
a/ Solubilisation de poudre de graines de *Avena strigosa* dans l'eau suivie de plusieurs hydrolyses enzymatiques (au moins deux hydrolyses) et d'inactivation enzymatique par traitement thermique
b/ Solubilisation de poudre de racines de *Ononis spinosa* dans un mélange Butylène Glycol/eau
c/ Mélange des produits issus des étapes a/ et b/,
d/ Séparation des phases soluble et insoluble, par exemple par décantation,
e/ Filtration(s) pour purifier le principe actif.

Le principe actif selon l'invention présente des caractéristiques qui permettent son utilisation en cosmétique et en particulier sur les cheveux pour un effet anti-grisonnement.

Le principe actif selon l'invention présente en effet avantageusement une action détoxifiante et protectrice, et ainsi favorise la repigmentation de la fibre capillaire et améliore la qualité structurale des cheveux. Ces effets biologiques se traduisent par une diminution significative du nombre de cheveux gris. Le principe actif selon l'invention, appliqué sur la chevelure, permet de diminuer la formation de radicaux libres et l'oxydation des acides aminés, et de détoxifier la cellule par activation de l'autophagie. En plus de cette action anti-radicalaire et détoxifiante, liée notamment à la présence de peptides spécifiques du principe actif, le principe actif selon l'invention présente préférentiellement également un effet pro-pigmentant des cheveux gris en couleur naturelle, liée notamment à la présence de composés phénoliques particuliers.

Les cheveux sont composés de deux structures anatomiques distinctes :
- la tige pilaire, partie visible qui émerge de la surface de la peau ;
- le follicule pileux sous-cutané.

C'est au sein du follicule pileux qu'interviennent les mécanismes à l'origine de la pigmentation du cheveu. Ainsi, la large variété de couleurs de cheveux est le résultat d'un mélange entre deux pigments nommés eumélanine (noir-brun) et phéomélanine (brun-rouge-jaune). Ces deux types de mélanine sont synthétisés par les mélanocytes, cellules spécialisées de la mélanogenèse, précisément dans les mélanosomes, des organites intracellulaires dédiés à ce phénomène biologique. Localisés au niveau de la papille dermique du follicule pileux, les mélanocytes transfèrent les mélanosomes aux kératinocytes pré-corticaux voisins qui se différencient et s'assemblent pour former la tige pilaire pigmentée en croissance.

Durant la phase de croissance, les différentes réactions enzymatiques nécessaires à la synthèse de mélanine génèrent de grandes quantités d'espèces réactives de l'oxygène (ROS) dont le peroxyde d'hydrogène (H2O2). Au-delà de ce stress radicalaire endogène, la synthèse de radicaux libres est également stimulée par certains facteurs environnementaux (rayonnements ultraviolets, pollution). Le follicule pileux est donc particulièrement exposé à l'accumulation de ces molécules hautement réactives qui peuvent endommager les lipides et protéines cellulaires. Ainsi, l'oxydation de certains acides aminés comme la méthionine altère les structures protéiques mais aussi le fonctionnement d'enzymes impliquées dans la mélanogenèse. Le stress oxydatif entraîne également une dégradation de la pigmentation via la mort des mélanocytes.

Dans ce contexte, des études ont depuis peu mis en avant l'intérêt de l'autophagie, mécanisme de détoxification cellulaire, dans la protection des mélanocytes. Ce processus est décrit pour sa capacité à protéger les mélanocytes du stress oxydatif en dégradant et en éliminant les protéines et organites non fonctionnels. L'autophagie se révèle être un mécanisme biologique majeur dans le maintien de l'homéostasie cellulaire au niveau du follicule pileux.

La mélanogenèse (ou synthèse de pigments) est un phénomène qui intervient dans les mélanocytes, des cellules spécialisées localisées au niveau du follicule pileux. En raison de la production de radicaux libres, elle est à l'origine d'un stress oxydatif dont l'intensité peut être aggravée par l'exposition à divers facteurs environnementaux. Ce déséquilibre oxydatif joue un rôle capital dans la dépigmentation du cheveu puisqu'il est à l'origine d'une inactivation des enzymes impliquées dans la mélanogénèse et/ou d'une induction de la mort des mélanocytes. Ainsi, pour s'en protéger, les cellules mettent en place une première ligne de défense capable d'éliminer les radicaux libres et de réparer les dégâts cellulaires occasionnés. En cas de dommages plus importants, une seconde ligne de défense impliquant l'autophagie conduit à l'élimination des éléments défaillants et donc à la détoxification cellulaire. Ces données récemment mises en évidence attestent du rôle important tenu par l'autophagie dans la défense du cheveu face au stress radicalaire. Si l'impact du stress oxydant au niveau cellulaire et son implication majeure dans le grisonnement du cheveu sont aujourd'hui compris, ses conséquences à l'échelle de la tige pilaire n'ont jamais été étudiées.

Avantageusement, le principe actif selon, l'invention présente un effet anti-radicalaire. De plus, au-delà de la prise en charge des radicaux libres, le principe actif selon l'invention est capable d'activer la détoxification cellulaire induite par l'autophagie. Ce mécanisme est un puissant processus capable de séquestrer et dégrader les protéines et organites intracellulaires endommagés. Ainsi, le principe actif selon l'invention rétablit l'équilibre oxydatif du cheveu en agissant sur deux niveaux de défense : la lutte contre les radicaux libres et l'autophagie, éléments incontournables de la stratégie anti-grisonnement.

De plus, les demandeurs ont démontré qu'au-delà de la problématique radicalaire il existe une modification des marqueurs moléculaires liés aux propriétés biomécaniques (conformation des protéines) et à la fonction barrière (organisation des lipides) dans le cheveu gris. En effet, ils ont étudié en microspectroscopie les modifications biologiques intervenant au cours du grisonnement en travaillant directement sur des échantillons de cheveux. Des mesures spectrales ont ainsi été réalisées sur trois types de cheveux : pigmentés, gris et non pigmentés. Le traitement bioinformatique des données a permis la classification des échantillons selon le degré de pigmentation des cheveux mais également l'identification de 3 stades de grisonnement intermédiaires invisibles à l'œil nu. Grâce à cette technologie, les demandeurs ont réalisé une analyse plus fine de l'évolution du grisonnement. Grâce à cette approche innovante, il a été décrit pour la première fois des changements intervenant dans la qualité structurale des cheveux au cours du grisonnement.

Avantageusement le principe actif selon l'invention est capable d'agir sur la qualité structurale des cheveux gris.et permet ainsi aux chevelures grisonnantes de retrouver leur couleur naturelle.

Ainsi, en agissant sur l'organisation des lipides et la conformation des protéines, deux paramètres en lien avec la fonction barrière et la mécanique des cheveux, le principe actif selon l'invention redonne force et résistance aux cheveux gris.

Le principe actif selon l'invention a donc une action multiple qui se traduit par un grisonnement de la chevelure qui devient moins perceptible et des cheveux qui retrouvent leur couleur naturelle et leur qualité structurale originelle.

L'invention a par conséquent pour objet l'utilisation cosmétique d'un principe actif cosmétique tel que précédemment décrit, seule ou dans une composition cosmétique, sur les cheveux prévenir et/ou lutter contre le grisonnement des cheveux, en particulier :
- pour préserver la pigmentation des cheveux et/ou
- pour améliorer la qualité structurale des cheveux, et/ou
- pour diminuer le nombre de cheveux gris.

Les principes actifs selon l'invention, sont de préférence utilisés dans des compostions cosmétiques comprenant un milieu cosmétiquement acceptable. Il s'agit de compositions dans différentes formes galéniques, préférentiellement adaptées à une application sur les cheveux.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, gels aqueux ou poudres. Elles peuvent être plus ou moins fluides et se présenter sous forme de shampoing, de gel capillaire, de lotion capillaire, de masque capillaire, de conditionneur capillaire, d'après-shampoing, de crème capillaire ou tous autres aspects des cosmétiques de soin des cheveux ou de coiffage.

Il peut s'agir de compositions comprenant au moins 0,1% du principe actif liquide selon l'invention, préférentiellement entre 0,5 et 10% ou comprenant au moins 0,01% d'un principe actif solide (poudre préférentiellement) selon l'invention.

Ces compositions comprennent, outre le principe actif, un milieu physiologiquement acceptable et de préférence cosmétiquement acceptable, c'est-à-dire qui ne provoque pas de sensations d'inconfort pour l'utilisateur telles que des rougeurs, tiraillements ou picotements du cuir chevelu.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles, huiles végétales et esters, beurres végétaux ;
- les cires, telles que l'ozokérite, la cire de polyéthylène, la cire d'abeille ou la cire de carnauba,
- les tensioactifs, qu'ils soient non ioniques, anioniques, cationiques ou amphotères,
- les co-émulsionnants et autres agents de consistance, tels que les alcools gras linéaires,
- les épaississants et/ou gélifiants,
- les humectants, tels que les polyols comme la glycérine,
- les colorants, les conservateurs, les charges,
- les séquestrants,
- l'alcool éthylique
- les agents conditionneurs, tels que le guar, les polymères cationiques,
- les vitamines : B5, B3, E, B6,
- les minéraux, tels que le zinc, le cuivre,
- les parfums,
- et leurs mélanges, sans que cette liste soit limitative.

Des exemples de tels adjuvants sont cités notamment dans le Dictionnaire CTFA (International Cosmetic Ingredient Dictionary and Handbook publié par le Personal Care Product Council). Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées sur des cheveux, notamment des cheveux gris ou des chevelures comportant des cheveux gris, en particulier pour un effet anti-grisonnement. L'invention a donc également pour objet un procédé cosmétique d'une chevelure d'un être humain, pour prévenir et/ou lutter contre le grisonnement des cheveux, qui consiste à appliquer au moins une fois par jour sur les cheveux une composition comprenant un principe actif selon l'invention.

Afin d'illustrer les effets cosmétiques sur les cheveux gris de principes actifs selon l'invention, des exemples et résultats d'essais sont présentés en suivant.

### Exemples

### Exemple 1 : exemple de principe actif selon l'invention sous forme liquide

Le principe actif est obtenu par la mise en œuvre des étapes suivantes :
a/mise en œuvre des étapes suivantes :
   - Solubilisation de graines *d'Avena strigosa* à raison de 100 g/L,
   - Hydrolyses à l'aide de deux protéases différentes, en suivant les instructions du fournisseur
   - Séparation des phases soluble et insoluble
   - Inactivation thermique
   - Filtrations
   - Désodorisation
   - Filtration
b/ mise en œuvre des étapes suivantes :
   - Solubilisation *d'Ononis spinosa à* 200g/L dans un mélange eau/butylène glycol 50/50 (vol/vol)
   - Agitation
   - Séparation des phases soluble et insoluble par décantation
   - Filtration
c/ Mélange des produits issus des étapes a/ et b/,
d/ Séparation des phases soluble et insoluble, par exemple par décantation
e/ Filtration(s) pour concentrer le principe actif

Le principe actif obtenu présente les caractéristiques analytiques suivantes :
- un taux de matières sèches de 26,6 g/L,
- une teneur en peptides de 30.5% en poids de matière sèche de l'hydrolysat (déterminée par la méthode de KJELDAHL)
- une teneur en sucres totaux de 39% en poids de matière sèche de l'hydrolysat (calculé à l'aide d'une gamme de glucose)
- une teneur en polyphénols de 21.4% en poids de matière sèche
- 8.3% de cendres en poids de matière sèche (déterminée par la pesée des résidus issus de l'incinération des échantillons de l'hydrolysat à 550°C dans un four à moufle électrique).

### Exemple 2 : exemple de composition de masque conditionneur selon l'invention

Un exemple de formulation contenant un principe actif selon l'invention est présenté dans le tableau 1 suivant. Ce masque est un soin capillaire à la texture onctueuse qui enveloppe les cheveux de douceur de la racine jusqu'aux pointes. Il peut être utilisé en masque de nuit, soin pré- ou après-shampoing. Après rinçage, les cheveux sont souples et brillants.

**[Tableau 1]**

| | Ingrédients | INCI name | % |
|---|---|---|---|
| A | Eau | Aqua (water) | qsp 100 |
| | Conservateur | - | qs |
| B | Incroquat behenyl TMS | Behentrimonium Methosulfate (and) Cetearyl Alcohol | 6,0 |
| | Alcool cétylique | Cetyl alcohol | 1,5 |
| | Crodamol SS | Cetyl esters | 3,0 |
| | Beurre de Karité | Butyrospermum parkii (shea) butter | 0,75 |
| | Bioxan SFT50 | Tocopherol & Helianthus annuus (sunflower) seed oil | 0,05 |
| C | Plantacare 818 UP | Coco-glucoside & aqua (water) | 1,0 |
| D | Principe actif de l'exemple 1 | Butylene glycol & Water *& Avena strigosa* seed extract *& Ononis spinosa* Root Extract | 2,5 |

Il est obtenu par la mise en œuvre du mode opératoire suivant :
- Placer A sous agitation magnétique et chauffer à 80°C.
- Placer B sous agitation magnétique et chauffer à 80°C.
- Sous rotor-stator, émulsionner B dans A.
- A 40°C, sous agitation modérée, ajouter C puis D.

### Exemple 3 : exemple de composition de shampoing anti-grisaillement selon l'invention

Un exemple de formulation contenant un principe actif selon l'invention est présenté dans le tableau 2 suivant. Ce shampoing développe une mousse onctueuse qui nettoie les cheveux en douceur.

**[Tableau 2]**

| | Ingrédients | INCI name | % |
|---|---|---|---|
| A | Eau purifiée | Aqua (water) | qsp 100 |
| B | Carbopol Aqua SF1 | Aqua (water) & Acrylates copolymer | 2,0 |
| C | Texapon N70 | Sodium laureth sulfate & aqua (water) | 10,3 |
| D | Conservateur | - | qs |
| E | Soude 28% | Sodium hydroxide & aqua (water) | qs pH 6,2 - 6,6 |
| F | Amonyl 675 SB | Cocamidopropyl Hydroxysultaine & aqua (water) | 5,0 |
| G | Rewoteric AM C | Sodium Cocoamphoacetate & aqua (water) | 8,0 |
| H | Oramix NS10 | Decyl glucoside & aqua (water) | 3,7 |
| I | Principe actif de l'exemple 1 | Butylene glycol & Water *& Avena strigosa* seed extract & *Ononis spinosa* Root Extract | 2,5 |
| J | Solution d'acide citrique 10% | Citric acid & aqua (water) | qs pH 5 - 5,5 |

Il est obtenu par la mise en œuvre du mode opératoire suivant :
- Disperser B dans A.
- Chauffer AB jusqu'à 70°C sous agitation magnétique.
- Ajouter C dans AB en cours de chauffe.
- A 70°C, ajouter D dans ABC.
- Refroidir à température ambiante sous agitation magnétique.
- Neutraliser avec E à pH 6,2 - 6,6.
- Ajouter F, G, H, et I.
- Ajuster le pH avec J à pH 5 - 5,5.

### Exemple 4 : exemple de composition de lotion capillaire selon l'invention

Un exemple de formulation contenant un principe actif selon l'invention est présenté dans le tableau 3 suivant. Non grasse et non collante, cette lotion sèche rapidement et laisse les cheveux souples sans les alourdir.

**[Tableau 3]**

| | Ingrédients | INCI name | % |
|---|---|---|---|
| A | Eau | Aqua (water) | qsp 100 |
| | Pentylène glycol | Pentylene glycol | 5,0 |
| | Butylène glycol | Butylene glycol | 10,0 |
| B | Ethanol | Alcohol | 15,0 |
| | Conservateur | - | qs |
| C | Principe actif de l'exemple 1 | Butylene glycol & Water *& Avena strigosa* seed extract & *Ononis spinosa* Root Extract | 2,5 |
| D | Solution acide citrique 10% | Citric Acid | qs pH 4,8- 5,2 |

Elle est obtenue par la mise en œuvre du mode opératoire suivant :
- Placer A sous agitation magnétique.
- Ajouter B puis C.
- Ajuster le pH entre 4,8 et 5,2 avec D.

### Exemple 5 : exemple de composition de gel Elixir de Jouvence selon l'invention

Un exemple de formulation contenant un principe actif selon l'invention est présenté dans le tableau 4 suivant. Il s'agit d'un gel ultra frais qui pénètre rapidement pour laisser les cheveux doux et soyeux sans résidus de gras.

**[Tableau 4]**

| | Ingrédients | INCI name | % |
|---|---|---|---|
| A | Eau | Aqua (water) | qsp 100 |
| | Pentylène glycol | Pentylene glycol | 5,0 |
| | Butylène glycol | Butylene glycol | 10,0 |
| B | Ethanol | Alcohol | 5,0 |
| | Conservateur | - | qs |
| C | Carbopol Ultrez 20 | Acrylates/C10-30 Alkyl cacrylate crosspolymer | 0,15 |
| D | Soude 28% | Sodium hydroxide & aqua (water) | qs pH 5 - 5,5 |
| E | Principe actif de l'exemple 1 | Butylene glycol & Water *& Avena strigosa* seed extract & *Ononis spinosa* Root Extract | 2,5 |

Il est obtenu par la mise en œuvre du mode opératoire suivant :
- Placer A sous agitation magnétique.
- Ajouter B dans A.
- Disperser C dans AB.
- Ajuster le pH entre 5,0 et 5,5 avec D.
- Ajouter E.

### Etude de l'efficacité du principe actif selon l'invention sur le grisonnement des cheveux

### A. Modélisation : établissement de la signature moléculaire du grisonnement des cheveux

L'objectif de cette étude est d'établir les caractéristiques moléculaires du cheveu gris.

La canitie est un phénomène progressif et naturel intervenant avec le temps. A la problématique de dépigmentation s'ajoute celle associée à la variation de la structure et du caractère hygroscopique du cheveu gris. La composition lipidique et la structure des protéines constituant les cheveux sont associées à la qualité structurale de la tige pilaire. À ce jour, les études sur les lipides et protéines capillaires ont principalement été réalisées en comparant les cheveux provenant de différents groupes ethniques ou en exposant les cheveux à des facteurs externes. Très peu d'études se sont intéressées à l'évolution de ces marqueurs moléculaires au cours du grisonnement.

Dans ce contexte, une étude a été réalisée par micro-spectroscopie Raman à la surface de cheveux prélevés in vivo, afin de déterminer les différentes modifications moléculaires intervenant dans les fibres non pigmentées, intermédiaires et pigmentées.

Le protocole opératoire est décrit en suivant.

L'analyse Raman a été réalisée sur 29 volontaires caucasiens, de sexe féminin ou masculin, âgés de 33 à 65 ans (âge moyen 52 ± 9 ans) et présentant un stade de grisonnement allant de 1 à 8 sur une échelle allant de 0 à 10 sur l'ensemble de la chevelure (zone frontale et temporale).

Pour chaque volontaire, les cheveux ont été prélevés (longueur d'environ 2 cm) et une sélection a été réalisée comme suit :
- 2 cheveux non pigmentés (blancs) ;
- 2 à 5 cheveux intermédiaires ;
- 2 cheveux pigmentés.

Les cheveux sont analysés par microspectromètre Raman.

Les mesures spectrales obtenues sont utilisées d'une part pour valider le type de cheveux par une classification hiérarchique, et d'autre part pour quantifier, par type de cheveux, les descripteurs moléculaires d'intérêt.

Afin de conforter le classement des différents types de cheveux définis visuellement, des groupes ont été constitués à l'aide d'une Classification Ascendante Hiérarchique (CAH) des spectres Raman. Sur la base des groupes définis précédemment, les spectres Raman moyens ont été calculés pour chaque groupe.

Les descripteurs d'intérêt sélectionnés sont :
- la quantité de mélanine ;
- la quantité d'hydrogène peroxydé ;
- l'oxydation des acides aminés ;
- la conformation des lipides ;
- la structure secondaire des protéines.

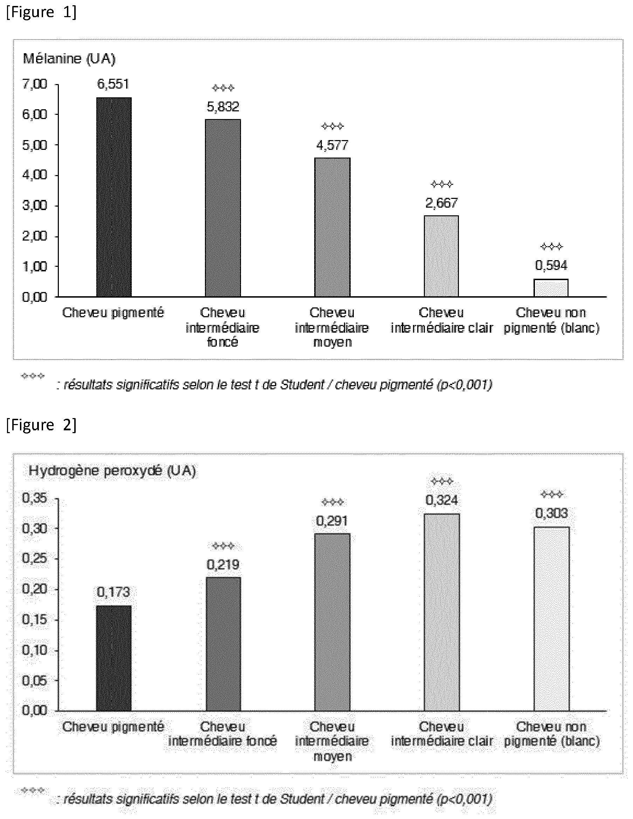

Tout d'abord une analyse de la quantité de mélanine a été effectuée. Cette analyse permet de déterminer la quantité de mélanine pour chaque groupe de cheveu établi selon la classification proposée précédemment. Les résultats sont présentés sur la Figure 1 qui représente une évolution de la mélanine en fonction de la pigmentation du cheveu.

Ces résultats montrent que la quantité de mélanine diminue avec la dépigmentation des cheveux. En effet, un certain nombre de facteurs sont impliqués dans l'altération progressive de la mélanogenèse, ce qui entraîne l'apparition progressive de fibres capillaires avec une faible teneur en pigment (cheveux gris) ou avec une absence totale de pigment (cheveux non pigmentés, blancs). Une analyse de l'oxydation des cheveux a également été effectuée : une analyse de l'hydrogène peroxydé et une analyse des acides aminés oxydés. La diminution progressive de la production de pigments est en grande partie causée par la présence d'espèces réactives de l'oxygène (ROS) au niveau du follicule pileux. L'accumulation d'hydrogène peroxydé contribue notamment à la mort des mélanocytes du follicule pileux. Ce stress oxydatif se mesure également à l'échelle de la tige pilaire, comme le démontre cette étude évaluant les molécules témoins de l'oxydation du cheveu. L'analyse de l'hydrogène peroxydé consiste à déterminer l'évolution de la quantité d'hydrogène peroxydé pour chaque groupe de cheveu établi selon la classification proposée précédemment. Les résultats sont présentés sur la figure 2 qui représente l'évolution de la quantité d'hydrogène peroxydé en fonction de la pigmentation du cheveu. On constate que la quantité d'hydrogène peroxydée augmente avec la dépigmentation du cheveu. Ces résultats démontrent l'accumulation de peroxyde d'hydrogène dans les cheveux gris et non pigmentés (blancs).

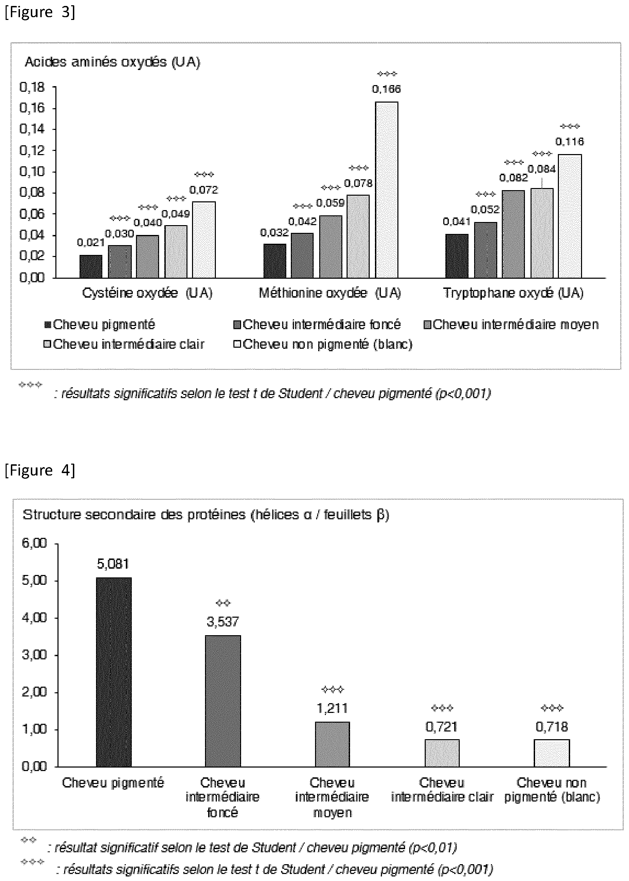

L'analyse des acides aminés oxydés consiste à déterminer l'évolution de la quantité d'acides aminés oxydés pour chaque groupe de cheveu établi selon la classification proposée précédemment. Les résultats sont présentés sur la figure 3 qui représente l'évolution des acides aminés oxydés en fonction de la pigmentation du cheveu. On constate que l'oxydation des protéines augmente avec la dépigmentation du cheveu. En effet, les acides aminés oxydés constituant la kératine, à savoir la cystéine, le tryptophane et la méthionine sont tous présents à des niveaux élevés dans les cheveux non pigmentés.

En plus de ces analyses, une analyse des marqueurs liés à la qualité structurale des cheveux a été effectuée, à savoir une analyse de la structure secondaire des protéines et une analyse de la conformation des lipides.

Tout d'abord une analyse de la structure secondaire des protéines (hélices α / feuillets β) a été réalisée. Les structures secondaires protéiques des cheveux pigmentés sont principalement sous forme d'hélices α ce qui leur confère la capacité à se déformer plastiquement sans se rompre (résistance/ductilité). L'analyse de la structure secondaire des protéines consiste à déterminer l'évolution de la structure secondaire protéique pour chaque groupe de cheveu établi selon la classification proposée précédemment. Les résultats sont présentés sur la figure 4 qui représente l'évolution du ratio hélices α / feuillets β en fonction de la pigmentation du cheveu. On constate que les structures secondaires protéiques en hélices α diminuent avec la dépigmentation du cheveu comparativement aux feuillets β qui augmentent. Ceci se traduit par une altération des propriétés biomécaniques des cheveux gris.

Une analyse de la conformation des lipides a été réalisée. Les lipides d'un cheveu pigmenté présentent principalement une conformation *trans.* Cette conformation permet une bonne organisation lipidique, un élément clé pour le maintien de l'imperméabilité et du caractère hydrophobe à la surface des fibres. La barrière lipidique capillaire est donc intimement liée à la conformation (compacité) lipidique dans la cuticule. L'analyse de la conformation des lipides permet de déterminer l'évolution de la conformation lipidique pour chaque groupe de cheveu établi selon la classification proposée précédemment. Les résultats sont présentés sur la figure 4 qui représente l'évolution du ratio *trans*/*gauche* en fonction de la pigmentation du cheveu. On constate que les lipides sous conformation *trans* diminuent avec la dépigmentation du cheveu tandis que ceux sous conformation *gauche* augmentent. Les changements de conformation lipidique observés avec la dépigmentation des cheveux témoignent d'une baisse de la fonction barrière au niveau de la fibre capillaire.

### B. Etude de l'effet anti-oxydant et détoxifiant du principe actif selon l'invention

L'objectif de cette étude est d'évaluer l'effet antioxydant et détoxifiant d'un principe actif selon l'invention par sa capacité à :
- limiter la production d'espèces réactives de l'oxygène (ROS) ;
- favoriser l'activité autophagique ;
- réduire l'oxydation des acides aminés de la fibre capillaire.

Le grisonnement des cheveux est perçu comme un signe de vieillissement et peut impacter de manière importante l'image que l'on renvoie. Bien que les mécanismes responsables de cette perte de pigmentation ne soient pas encore totalement élucidés, ce grisonnement semble être dû à un dysfonctionnement et une raréfaction des mélanocytes du follicule pileux pouvant être attribués aux effets néfastes des ROS. En effet, les mélanocytes folliculaires sont en permanence soumis à un haut niveau de stress oxydatif lié à la mélanogénèse et à leur exposition quotidienne à des facteurs environnementaux tels que la pollution ou les UV. Or, avec l'âge, leur capacité à faire face à cette oxydation s'amoindrit. Différentes études ont souligné un affaiblissement du système anti-oxydant et une accumulation progressive de ROS notamment de peroxyde d'hydrogène (H₂O₂) dans les follicules pileux gris.

Parmi les processus mis en place par la cellule pour lutter contre le stress oxydatif, l'autophagie tient un rôle majeur en permettant l'élimination des organites endommagés. Dans ce sens, une étude a révélé l'importance de l'autophagie dans la protection des mélanocytes face aux dommages induits par une exposition à l'H₂O₂.

Lutter contre le stress oxydatif et ses méfaits (ie oxydation des acides aminés) dans le follicule pileux en limitant la production de ROS et en favorisant l'activation de l'autophagie peut être considéré comme une stratégie d'intérêt et novatrice pour contrecarrer le grisonnement des cheveux.

Pour cette étude, différents modèles ont été mis en place et différents paramètres ont été évalués :
- la production de ROS et l'activité de l'autophagie ont été quantifiées à l'aide de sondes fluorescentes spécifiques respectivement analysées par cytométrie en flux ou microscopie à confocale sur des mélanocytes humains soumis à un stress oxydatif ;
- les quantités d'H₂O₂ et d'acides aminés oxydés ont été évaluées par microspectroscopie Raman sur cheveux issus de volontaires.

Le protocole opératoire pour l'analyse de la production de ROS par les mélanocytes humains est décrit en suivant.

A J0, les mélanocytes humains sont ensemencés et incubés à 37°C dans une atmosphère contenant 5% de CO₂.

A J1, les mélanocytes humains sont traités avec le principe actif de l'exemple 1 à 0,025% et 0,050% (V/V).

A J2, les mélanocytes humains sont récupérés et incubés 30 minutes avec la sonde DCFDA. Les mélanocytes sont traités avec une solution d'H₂O₂ en présence du principe actif selon l'invention à 0,025% et 0,050%(V/V).

La production de ROS par les mélanocytes est ensuite analysée par cytométrie en flux.

La production de ROS est proportionnelle à l'intensité de la fluorescence présente dans les cellules. Une analyse quantitative a été effectuée à l'aide d'un cytomètre en flux (FC500 MPL, Beckman Coulter). L'acquisition et l'analyse sont réalisées à l'aide des logiciels MXP acquisition et CXP analysis (Beckman Coulter). L'intensité moyenne de fluorescence est exprimée en unités arbitraires (UA).

Les résultats sont donnés dans le tableau 5.

**[Tableau 5]**

| | Quantité de ROS (UA) | | Capacité à limiter la production de ROS (%) |
|---|---|---|---|
| Mélanocytes humains normaux | | | |
| Témoin | 13,16 | | |

| Mélanocytes humains soumis à un stress oxydatif | | | |
|---|---|---|---|
| Témoin | 40,07^^^ | | |
| Exemple 1 à 0,025% | 37,18*** | | 11 |
| Exemple 1 à 0,050% | 33,47*** | | 25 |

| | | | |
|---|---|---|---|
| ^^^ : résultat significatif selon le test t de Student / mélanocytes normaux témoin (p < 0,001) *** : résultats significatifs selon le test t de Student / mélanocytes stressés témoin (p < 0,001) | | | |

Le protocole opératoire pour l'analyse de l'activation de l'autophagie par les mélanocytes humains est décrit en suivant.

A J0, les mélanocytes humains sont ensemencés et incubés à 37°C dans une atmosphère contenant 5% de CO₂.

A J2, les mélanocytes humains sont traités avec une solution d'H₂O₂. Après stress, les mélanocytes sont traités avec le principe actif de l'exemple 1 à 0,025% et 0,050%(V/V).

Ensuite, les mélanocytes humains sont marqués avec une sonde fluorescente permettant de détecter les vésicules d'autophagie.

Le niveau d'autophagie est proportionnel à l'intensité de la fluorescence verte présente dans les cellules. Une analyse quantitative des images a été réalisée à l'aide du logiciel Matlab^{®}. L'intensité moyenne de fluorescence par cellule est exprimée en unités arbitraires (UA).

Les résultats sont donnés dans le tableau 6.

**[Tableau 6]**

| | Quantité de vésicules d'autophagie (UA) | | Capacité à activer l'autophagie (%) |
|---|---|---|---|
| Mélanocytes humains normaux | | | |
| Témoin | 919 | | |

| Mélanocytes humains soumis à un stress oxydatif | | | |
|---|---|---|---|
| Témoin | 4395^^ | | |
| Exemple 1 à 0,025% | 5306 | | 21 |
| Exemple 1 à 0,050% | 7773* | | 77 |

| | | | |
|---|---|---|---|
| ^^ : résultat significatif selon le test t de Student / mélanocytes normaux témoin (p < 0,01) * : résultat significatif selon le test t de Student / mélanocytes stressés témoin (p < 0,05) | | | |

Le protocole opératoire pour l'analyse de la quantité d'H₂O₂ et d'acides aminés oxydés sur cheveux issus de volontaires est décrit en suivant.

L'analyse Raman a été réalisée sur 10 volontaires caucasiens, de sexe féminin ou masculin, âgés de 33 à 65 ans (âge moyen 50 ± 10 ans), présentant un stade de grisonnement allant de 2 à 8 sur une échelle allant de 0 à 10 sur l'ensemble de la chevelure (zone frontale et temporale). Les volontaires ont appliqué le principe actif de l'exemple 1 formulé à 2,5% dans une lotion, pendant 6 mois.

Les cheveux prélevés (longueur d'environ 2 cm) sont analysés.

La capacité du principe actif selon l'invention à réduire le stress oxydatif à l'échelle de la tige pilaire a été évaluée à l'aide des descripteurs suivants :
- la bande à 875 cm⁻¹ est utilisée pour quantifier l'hydrogène peroxydé (H2O2) présente dans le cheveu par spectroscopie Raman. Des valeurs élevées de cette bande sont associées à un stress oxydatif élevé.
- les bandes S-O sont témoin de l'oxydation des acides aminés soufrés (cystéine oxydée : 1030 cm-1, méthionine oxydée : 1040 cm-1) et du tryptophane oxydé (kynurénine) : 1050 cm-1. Le blanchissement du cheveu montre une augmentation de ces bandes.

Les résultats obtenus montrent, après 4 mois d'application du principe actif selon l'invention :
- une diminution de 18% de la quantité globale d'hydrogène peroxydé contenue dans les cheveux,
- une diminution de 31% de cystéine oxydée contenue dans les cheveux,
- une diminution de 35% de méthionine oxydée contenue dans les cheveux, et
- une diminution de 26% de tryptophane oxydé contenue dans les cheveux.

Les résultats obtenus montrent, après 6 mois d'application du principe actif selon l'invention :
- une diminution de 19% de la quantité globale d'hydrogène peroxydé contenues dans les cheveux,
- une diminution de 31% de cystéine oxydée contenue dans les cheveux,
- une diminution de 36% de méthionine oxydée contenue dans les cheveux, et
- une diminution de 30% de tryptophane oxydé contenue dans les cheveux.

Ainsi l'ensemble de ces résultats montrent que :
- testé à 0,050% sur mélanocytes humains soumis à un stress oxydatif, le principe actif selon l'invention limite significativement la production de ROS de 25% et active l'autophagie de 77%.
- après 4 mois d'application, le principe actif selon l'invention est formulé à 2,5% diminue significativement, dans la tige pilaire, les quantités d'hydrogène peroxydé (-18%) et d'acides aminés oxydés (cystéine oxydée : -31% ; méthionine oxydée: -35% et tryptophane oxydé : -26%).

Cet effet se maintient après 6 mois d'application quotidienne.

L'ensemble de ces données met en évidence le fort potentiel anti-oxydant et détoxifiant du principe actif selon l'invention.

### C. Etude de l'effet cryoprotecteur du principe actif selon l'invention

L'objectif de cette étude est d'évaluer la capacité d'un principe actif selon l'invention à maintenir la viabilité des mélanocytes humains suite à un stress oxydatif.

Le grisonnement est caractérisé par une perte de pigments dans la tige pilaire qui peut en partie être attribuée à une raréfaction des mélanocytes folliculaires. En effet, la canitie s'accompagne d'une production excessive de radicaux libres par les mélanocytes, un phénomène pouvant compromettre leur viabilité.

Préserver la viabilité des mélanocytes face à un stress oxydatif est une stratégie de choix pour éviter leur déclin avec l'âge et lutter contre le grisonnement des cheveux.

La viabilité des mélanocytes humains suite à un stress oxydatif a été évaluée par cytométrie en flux. Le protocole opératoire de l'étude est décrit en suivant.

A J0, les mélanocytes humains sont ensemencés et incubés à 37°C dans une atmosphère contenant 5% de CO₂.

A J1, les mélanocytes humains sont traités avec le principe actif de l'exemple 1 à 0,025% et 0,050%(V/V).

A J2, les mélanocytes humains sont traités avec une solution d'H₂O₂ en présence du principe actif de l'exemple 1 à 0,025% et 0,050%(V/V).

Après le stress, les mélanocytes sont de nouveau traités pendant 24 heures avec le principe actif de l'exemple 1 à 0,025% et 0,050%(V/V).

A J3, les mélanocytes sont récupérés et incubés avec de l'annexine V couplée FITC et de l'iodure de propidium.

La viabilité des mélanocytes est analysée par cytométrie en flux grâce à un double marquage annexine V / iodure de propidium.

Ces deux réactifs permettent de faire la distinction entre des cellules viables et des cellules progressant dans des voies de mort cellulaire (apoptose, nécrose) :
- l'apoptose est mise en évidence par fixation spécifique de l'annexine V couplée à un fluorophore. Dans une cellule saine, les phosphatidylsérines ne sont situées que sur la face interne de la membrane plasmique. Dès qu'une cellule entre en apoptose, elles sont délocalisées et exprimées des deux côtés de la membrane. L'annexine V est capable de marquer les cellules apoptotiques de par sa forte affinité pour les phosphatidylsérines.
- l'iodure de propidium (IP) est un agent intercalant de l'ADN capable de marquer le noyau des cellules ayant perdu leur intégrité membranaire. Ce phénomène est caractéristique de la nécrose ou de l'apoptose tardive.

Les mélanocytes viables ne sont pas marqués : ils sont négatifs pour le marquage à l'annexine V et pour le marquage à l'IP.

Le pourcentage de mélanocytes viables est mesuré à l'aide d'un cytomètre en flux (FC500 MPL, Beckman Coulter). Pour chaque condition, un nombre identique d'événement est analysé (10 000 cellules). Le pourcentage de mélanocytes viables correspond à la proportion de mélanocytes négatifs pour l'annexine V et l'IP par rapport à l'effectif total.

L'acquisition et l'analyse sont réalisées à l'aide des logiciels MXP acquisition et CXP analysis (Beckman Coulter).

Les résultats sont présentés dans le tableau 7.

**[Tableau 7]**

| | Mélanocytes viables (%) | | Capacité à protéger les mélanocytes (%) |
|---|---|---|---|
| Mélanocytes humains normaux | | | |
| Témoin | 86,83 | | |

| Mélanocytes humains soumis à un stress oxydatif | | | |
|---|---|---|---|
| Témoin | 66,78^{^^^} | | |
| Exemple 1 à 0,025% | 77,14 | | 52 |
| Exemple 1 à 0,050% | 84,48*** | | 88 |

| | | | |
|---|---|---|---|
| ^{^^^} : résultat significatif selon le test t de student / mélanocytes normaux témoin (p < 0,001) *** : résultat significatif selon le test t de student / mélanocytes stressés témoin (p < 0,001) | | | |

On constate que testé à 0,050% face à un stress oxydatif, le principe actif selon l'invention préserve significativement la viabilité des mélanocytes de 88% et présente ainsi un effet cytoprotecteur.

### D. Etude de l'effet pigmentant du principe actif selon l'invention

L'objectif de cette étude est d'évaluer la capacité d'un principe actif selon l'invention à stimuler la production de mélanine.

Les mélanocytes responsables de la pigmentation des cheveux sont localisés dans le bulbe des follicules pileux. Ces mélanocytes produisent et transfèrent la mélanine sous forme de mélanosomes aux kératinocytes pré-corticaux de la tige pilaire. La perception des cheveux gris provient de l'effet produit par le mélange de cheveux pigmentés et non pigmentés. Néanmoins les fibres capillaires individuelles peuvent présenter une dilution progressive de leurs pigments et donc différents niveaux de grisonnement. Cette dilution est liée à une réduction de la production de mélanine, causée notamment par l'altération d'enzymes impliquées dans la mélanogenèse ou par la mort des mélanocytes.

La production de la mélanine a été évaluée par :
- dosage sur mélanocytes humains soumis à un stress oxydatif ;
- immunohistochimie sur follicules pileux *ex vivo* (coloration Warthin-Starry) ;
- microspectroscopie Raman sur cheveux issus de volontaires caucasiens.

Le protocole opératoire de l'étude pour l'analyse de la production de mélanine par les mélanocytes humains est décrit en suivant.

A J0, les mélanocytes humains sont ensemencés et incubés à 37°C dans une atmosphère contenant 5% de CO₂.

A J1, les mélanocytes humains sont traités avec le principe actif de l'exemple 1 à 0,025% et 0,050% (V/V).

A J3, les mélanocytes humains sont traités avec une solution d'H₂O₂ en présence du principe actif de l'exemple 1 à 0,025% et 0,050%(V/V).

Après le stress, les mélanocytes sont traités de nouveau pendant 24 heures avec le principe actif de l'exemple 1 à 0,025% et 0,050%(V/V).

A J4, les cellules sont récupérées, comptées en vue du dosage de la mélanine.

Les culots cellulaires sont lysés dans un tampon sodium phosphate additionné de Triton X-100. Les lysats cellulaires sont centrifugés puis dissous dans une solution de NaOH pour permettre le dosage de la mélanine. Le taux de mélanine est ensuite quantifié par lecture spectrophotométrique à 490 nm grâce à un spectrophotomètre.

Les résultats sont donnés dans le tableau 8.

**[Tableau 8]**

| | Production de mélanine (µg/10⁶ cellules) | | Capacité à augmenter la production de mélanine (%) |
|---|---|---|---|
| Mélanocytes humains normaux | | | |
| Témoin | 32,44 | | |

| Mélanocytes humains soumis à un stress oxydatif | | | |
|---|---|---|---|
| Témoin | 23,93^^^ | | |
| Exemple 1 à 0,025% | 26,66* | | 32 |
| Exemple 1 à 0,050% | 34,76*** | | 127 |

| | | | |
|---|---|---|---|
| ^^^ : résultat significatif selon le test des rangs signés / mélanocytes normaux témoin (p < 0,001) * : résultat significatif selon le test des rangs signés / mélanocytes stressés témoin (p < 0,05) *** : résultat significatif selon le test des rangs signés / mélanocytes stressés témoin (p < 0,001) | | | |

Le protocole opératoire de l'étude pour l'analyse de la production de mélanine dans les follicules pileux *ex vivo* est décrit en suivant.

Cette étude a été réalisée selon le modèle de culture de follicules pileux *ex vivo* développé par Philpott *et al.* La capacité d'un principe actif selon l'invention à stimuler la production de la mélanine a été évaluée par coloration Warthin-Starry. Cette analyse a exclusivement été réalisée sur des follicules pileux en phase anagène. La phase anagène des follicules pileux a été contrôlée via l'analyse de la synthèse de Ki-67 par immunohistofluorescence. Cette étude a été conduite sur des follicules pileux provenant de fragments de cuir chevelu issus de 2 donneurs (23 et 33 ans).

A J0, Les follicules pileux sont isolés à partir de fragments de cuir chevelu et incubés dans du milieu de culture à 37°C dans une étuve contenant 5% de CO₂.

A J1, Les follicules pileux sont photographiés et mesurés sur microscope IX 70 (Olympus), couplé à un système d'analyse d'images (logiciel NIS-Elements, Nikon). Les follicules pileux en phase anagène sont sélectionnés et traités avec le principe actif selon l'invention de l'exemple 1 à 0,10% (V/V final). A J4, les follicules pileux sont photographiés à l'aide d'un microscope IX 70 (Olympus) couplé à un système d'analyse d'images (logiciel NIS-Elements, Nikon).

Les follicules sont ensuite récupérés et inclus en Tissue-Tek^{®} puis congelés. Des coupes (6 µm) sont réalisées à l'aide d'un cryostat CM1850 (Leica) puis fixées.

Une coloration Warthin-Starry est réalisée afin de visualiser le contenu en mélanine des follicules pileux. La mélanine correspond aux grains noirs présents sur les coupes après coloration.

Cette coloration est réalisée avec le kit Warthin--Starry staining kit (Abcam) en suivant les instructions du fournisseur.

Les cellules du follicule pileux sont mises en évidence par une coloration rose avec une solution de nuclear fast red (Abcam) en suivant les instructions du fournisseur.

Le contenu en mélanine a été analysé dans des régions d'intérêt (ROI) standardisées et adaptées à chaque follicule. Les repères morphologiques servant au positionnement des ROI sont : la ligne d'auber (ligne virtuelle traversant la papille dermique au niveau de sa partie la plus large) et la jonction entre la gaine épithéliale externe et la gaine conjonctive. Figure 19. Schéma du traitement d'images réalisé pour l'analyse de la quantité de mélanine sur follicules pileux par coloration Whartin-Starry.

Une analyse quantitative automatique a été réalisée à l'aide du logiciel Matlab^{®}. Le contenu en mélanine correspond à la surface occupée par la mélanine sur la surface totale de la zone de référence. Le contenu en mélanine est exprimé en unités arbitraires (UA).

Les résultats sont donnés dans le tableau 9.

**[Tableau 9]**

| | Contenu en mélanine (UA) | Augmentation de production de mélanine (%) |
|---|---|---|
| Follicules pileux humains ex vivo | | |
| Témoin | 0,186 | |
| Exemple 1 à 0,10% | 0,257* | 38 |

| | | |
|---|---|---|
| * : résultat significatif selon le test t de Student / follicules pileux témoin (p < 0,05) | | |

Le protocole opératoire de l'étude pour l'analyse de la quantité de mélanine sur cheveux issus des volontaires caucasiens est décrit en suivant.

L'analyse Raman a été réalisée sur 10 volontaires caucasiens, de sexe féminin ou masculin, âgés de 33 à 65 ans (âge moyen 50 ± 10 ans), présentant un stade de grisonnement allant de 2 à 8 sur une échelle allant de 0 à 10 sur l'ensemble de la chevelure (zone frontale et temporale). Les volontaires ont appliqué le principe actif de l'exemple 1 formulé à 2,5% dans une lotion, pendant 6 mois.

Les cheveux prélevés (longueur d'environ 2 cm) sont analysés avec le microspectromètre Raman (LabRam Evolution, Horiba).

La capacité du principe actif selon l'invention à réduire la quantité de mélanine a été évaluée à l'aide du descripteur suivant : la bande à 1580 cm⁻¹ est utilisée pour quantifier la mélanine présente dans le cheveu par spectroscopie Raman. Des valeurs élevées de cette bande sont associées à une grande quantité de mélanine.

On constate une augmentation globale de la mélanine contenue dans les cheveux issus de volontaires de 41% après 4 mois de traitement et de 40% après 6 mois de traitement.

Ainsi l'ensemble de ces résultats montrent que :
- Testé à 0,050% sur des mélanocytes humains soumis à un stress oxydatif, le principe actif selon l'invention augmente significativement la production de mélanine de 127%,
- Testé à 0,10% sur des follicules pileux humains, le principe actif selon l'invention stimule significativement la production de mélanine de 38%.
- Après 4 mois d'application, le principe actif selon l'invention formulé à 2,5% en lotion augmente significativement la quantité de mélanine dans les cheveux gris de 41%.

Cet effet se maintient après 6 mois d'application quotidienne (+40%)

L'ensemble de ces données met en évidence l'effet pigmentant d'un principe actif selon l'invention.

### E. Etude de l'effet du principe actif selon l'invention sur la qualité structurale du cheveu

L'objectif de cette étude a été d'évaluer l'effet d'un principe actif selon l'invention sur les structures secondaires protéiques et la conformation des lipides, dans des cheveux caucasiens prélevés après 4 et 6 mois de traitement quotidien.

Sur le plan moléculaire, la tige pilaire se compose principalement de protéines de type kératine, de lipides, de mélanine et d'eau.

Les protéines de type kératine s'organisent en faisceaux constituant le cortex capillaire. Les hélices α. sont avec les feuillets β des exemples de structures secondaires déterminant localement la conformation spatiale de la chaîne peptidique. A l'échelle du cheveu, les protéines sont principalement en hélices α , une structure qui contribue aux propriétés physiques et mécaniques ainsi qu'à la stabilité structurelle de la fibre capillaire. Dans les cheveux gris, le rapport hélice α / feuillet β diminue ce qui se traduit par une altération des propriétés biomécaniques des cheveux. Les lipides jouent également un rôle fondamental dans le cheveu puisqu'ils constituent une matrice dont l'organisation spécifique maintient l'étanchéité de la cuticule. Les lipides sous conformation *trans* assurent la fonctionnalité de la barrière capillaire. Dans les cheveux gris, le rapport *trans* / *gauche* diminue, ce qui traduit une désorganisation lipidique ainsi que la dégradation de la fonction barrière capillaire.

Le protocole opératoire de l'étude est décrit en suivant.

L'analyse Raman a été réalisée sur 10 volontaires caucasiens, de sexe féminin ou masculin, âgés de 33 à 65 ans (âge moyen 50 ± 10 ans), présentant un stade de grisonnement allant de 2 à 8 sur une échelle allant de 0 à 10 sur l'ensemble de la chevelure (zone frontale et temporale). Les volontaires ont appliqué un principe actif selon l'invention formulé à 2,5% dans une lotion, pendant 6 mois. Les cheveux prélevés (longueur d'environ 2 cm) sont analysés avec le microspectromètre Raman (LabRam HR Evolution, Horiba).

Pour chaque volontaire et à chaque temps de mesure :
- 15 cheveux sont triés au hasard ;
- 5 points de mesures Raman sont réalisés à la surface de chaque cheveu.

La capacité d'un principe actif selon l'invention à augmenter le rapport hélices α / feuillets β des protéines et le rapport *trans* / gauche des lipides a été évaluée à l'aide des descripteurs suivants :
- Le rapport hélices α / feuillets β (1650/1670 cm⁻¹) donne des informations sur la structure secondaire des protéines. La prédominance de la conformation structurale en hélice α est reliée à une bonne résistance de la fibre capillaire aux déformations mécaniques.
- Le rapport vCC *trans* / vCC gauche (1130/1080 cm⁻¹) donne des informations sur la conformation intramoléculaire des lipides. La région spectrale des élongations CC (1080-1130 cm-1) donne des informations sur la conformation des lipides. La prédominance de la conformation *trans* est reliée à une compacité élevée de la barrière lipidique. En revanche, une quantité élevée de conformères gauche, traduit un affaiblissement de la compacité des édifices lipidiques du cheveu

Les résultats correspondant à l'effet du principe actif de l'exemple 1 formulé à 2,5% en lotion, sur le rapport hélice α / feuillet β des protéines après 4 mois de traitement montrent une augmentation de 34% et après 6 mois de traitement montrent une augmentation de 37%.

Les résultats correspondant à l'effet du principe actif de l'exemple 1, formulé à 2,5% en lotion, sur le rapport *trans* / gauche des lipides après 4 mois de traitement quotidien montrent une augmentation de 25% et après 6 mois de traitement montrent une augmentation de 33%.

En augmentant la prédominance des structures secondaires en hélices α (reliée à une meilleure organisation des édifices protéiques sur le plan mécanique) et des lipides en conformation *trans* (reliée à une compacité plus élevée des édifices lipidiques et une bonne fonction barrière), le principe actif selon l'invention améliore la qualité structurale des cheveux.

### F. Etude de l'effet du principe actif selon l'invention sur la proportion de cheveux gris

Lors de cette étude, l'effet in vivo d'un principe actif selon l'invention formulé à 2,5% dans une lotion, sur l'évolution de la proportion de cheveux gris de sujets caucasiens et asiatiques a été évalué après 4 et 6 mois de traitement quotidien.

Le panel caucasien était composé 32 volontaires sains, de sexe féminin ou masculin, âgés de 33 à 65 ans (âge moyen 52 ± 9 ans), et présentant un stade de grisonnement allant de 1 à 8 sur une échelle allant de 0 à 10 sur l'ensemble de la chevelure (zone frontale et temporale).

Le panel asiatique était constitué de 17 volontaires sains, de sexe féminin ou masculin, âgés de 37 à 61 ans (âge moyen 51 ± 8 ans), présentant un stade de grisonnement compris entre 2 et 5 d'une échelle allant de 1 à 9 et une canitie installée depuis au moins 10 ans.

L'ensemble des volontaires a appliqué le principe actif selon l'invention pendant 6 mois, uniquement le soir, sur l'ensemble de la chevelure et du cuir chevelu.

La capacité du principe actif selon l'invention à réduire la proportion de cheveux gris a été évaluée, aux différents temps de l'étude, à l'aide des méthodes suivantes :
1/ Sur panel caucasien :
   - Comptage des cheveux gris sur des acquisitions dermatoscopiques du cuir chevelu réalisées à l'aide d'un Trichoscan HD (Tricholog GmbH).
   - Notation en aveugle du stade de grisonnement sur des photographies réalisées sur la zone frontale par un jury d'experts à l'aide d'une échelle de score (11 stades allant de 0 à 10).
2/ Sur panel asiatique :
   - Notation par scorage clinique de la densité des cheveux blancs par un dermatologue directement sur volontaires, à l'aide d'une échelle de score (Stade 1 à 9).

Les résultats obtenus sur le panel caucasien sont présentés en suivant.

Les résultats de la notation à l'aveugle montrent qu'après 4 mois d'application quotidienne, le principe actif selon l'invention formulé à 2,5% en lotion réduit significativement le nombre de cheveux gris de 9% (p = 0,0068). Cet effet se prolonge après 6 mois d'application pour atteindre une diminution de 12% (p = 0,0045), soit une diminution moyenne de 8 cheveux gris / cm².

Les résultats de la notation par scorage clinique montrent qu'après 4 mois d'application quotidienne, le principe actif selon l'invention formulé à 2,5% en lotion diminue significativement le stade de grisonnement de 20% (p < 0,001), correspondant à une diminution moyenne de 1 stade de grisonnement. 91% des volontaires ont montré une réduction du stade de grisonnement. Cet effet se maintient après 6 mois d'application quotidienne, avec une diminution de 18% (p < 0,001). Une amélioration du stade de grisonnement a été observée chez 94% des volontaires.

Les résultats obtenus sur le panel asiatique sont présentés en suivant.

Les résultats de la notation à l'aveugle montrent qu'après 4 mois d'application quotidienne, le principe actif selon l'invention formulé à 2,5% en lotion diminue significativement le stade de grisonnement de 7% (p = 0,0359). Cet effet se maintient après 6 mois d'application quotidienne, avec une diminution de 7% (p = 0,0359).

## Revendications

1. Principe actif cosmétique comprenant au moins un extrait *d'Avena strigosa* et au moins un extrait *d'Ononis spinosa.*

2. Principe actif cosmétique selon la revendication 1, **caractérisé en ce qu'**il comprend au moins un extrait de graines *d'Avena strigosa* et au moins un extrait de racines *d'Ononis spinosa.*

3. Principe actif cosmétique selon l'une des précédentes revendications, **caractérisé en ce qu'**il comprend au moins des peptides.

4. Principe actif cosmétique selon l'une des précédentes revendications, **caractérisé en ce qu'**il comprend au moins des peptides de masses molaires inférieures à 2000 Da, lesdits peptides représentent au moins 30% en poids de matière sèche du principe actif.

5. Principe actif cosmétique selon l'une des précédentes revendications, **caractérisé en ce qu'**il comprend au moins des composés phénoliques.

6. Principe actif cosmétique selon l'une des précédentes revendications, **caractérisé en ce qu'**il comprend au moins des acides phénoliques et des flavonoïdes, lesdits acides phénoliques et flavonoïdes représentent au moins 22% en poids de matière sèche du principe actif.

7. Principe actif cosmétique selon l'une des précédentes revendications, **caractérisé en ce que** l'extrait *d'Avena strigosa* est un hydrolysat *d'Avena strigosa.*

8. Principe actif cosmétique selon l'une des précédentes revendications, **caractérisé en ce que** l'extrait *d'Ononis spinosa* est un extrait hydroglycolique *d'Ononis spinosa.*

9. Principe actif cosmétique selon l'une des précédentes revendications, **caractérisé en ce que** le ratio extrait *d'Avena strigosa* / extrait *d'Ononis spinosa* est compris entre 45/55 et 55/45 en volume.

10. Composition cosmétique comprenant au moins 0,1% en poids d'un principe actif selon l'une des revendications 1 à 9, **caractérisée en ce qu'**elle se présente sous forme de shampoing, de gel capillaire, de lotion capillaire, de masque capillaire, de conditionneur capillaire, d'après-shampoing, de crème capillaire.

11. Procédé d'obtention d'un principe actif selon l'une des revendications 1 à 9, **caractérisé en ce qu'**il comprend les étapes suivantes :
a/ Solubilisation de poudre de graines de *Avena strigosa* dans l'eau suivie d'hydrolyses enzymatiques et d'inactivation enzymatique par traitement thermique,
b/ Solubilisation de poudre de racines de *Ononis spinosa* dans un mélange Butylène Glycol/eau,
c/ Mélange des produits issus des étapes a/ et b/
d/ Séparation des phases soluble et insoluble,
e/ Filtration(s).

12. Utilisation cosmétique d'un principe actif cosmétique selon l'une des revendications 1 à 9 sur les cheveux prévenir et/ou lutter contre le grisonnement des cheveux.

13. Utilisation cosmétique d'une composition cosmétique selon la revendication 10, sur les cheveux pour prévenir et/ou lutter contre le grisonnement des cheveux.

14. Utilisation cosmétique selon l'une des revendications 12 ou 13, pour préserver la pigmentation des cheveux et/ou pour améliorer la qualité structurale des cheveux et/ou pour diminuer le nombre de cheveux gris.

15. Procédé de traitement cosmétique d'une chevelure d'un être humain, pour prévenir et/ou lutter contre le grisonnement des cheveux, **caractérisé en ce qu'**il consiste à appliquer au moins une fois par jour sur les cheveux une composition selon la revendication 10.

## Patentansprüche

1. Kosmetischer Wirkstoff, umfassend mindestens einen Extrakt von *Avena strigosa* und mindestens einen Extrakt von *Ononis spinosa.*

2. Kosmetischer Wirkstoff nach Anspruch 1,
**dadurch gekennzeichnet, dass** er mindestens einen Samenextrakt von *Avena strigosa* und mindestens einen Wurzelextrakt von *Ononis spinosa* umfasst.

3. Kosmetischer Wirkstoff nach einem der vorstehenden Ansprüche, **dadurch gekennzeichnet, dass** er mindestens Peptide umfasst.

4. Kosmetischer Wirkstoff nach einem der vorstehenden Ansprüche, **dadurch gekennzeichnet, dass** er mindestens Peptide mit Molmassen von weniger als 2000 Da umfasst, wobei diese Peptide mindestens 30 Gew.-% der Trockenmasse des Wirkstoffs darstellen.

5. Kosmetischer Wirkstoff nach einem der vorstehenden Ansprüche,
**dadurch gekennzeichnet, dass** er mindestens phenolische Verbindungen umfasst.

6. Kosmetischer Wirkstoff nach einem der vorstehenden Ansprüche, **dadurch gekennzeichnet, dass** er mindestens Phenolsäuren und Flavonoide umfasst, wobei die Phenolsäuren und Flavonoide mindestens 22 Gew.-% der Trockenmasse des Wirkstoffs darstellen.

7. Kosmetischer Wirkstoff nach einem der vorstehenden Ansprüche, **dadurch gekennzeichnet, dass** der Extrakt von *Avena strigosa* ein Hydrolysat von *Avena strigosa* ist.

8. Kosmetischer Wirkstoff nach einem der vorstehenden Ansprüche, **dadurch gekennzeichnet, dass** der Extrakt von *Ononis spinosa* ein hydroglykolischer Extrakt von *Ononis spinosa* ist.

9. Kosmetischer Wirkstoff nach einem der vorstehenden Ansprüche,
**dadurch gekennzeichnet, dass** das Verhältnis von dem Extrakt von *Avena strigosa* zu dem Extrakt von *Ononis spinosa* zwischen 45:55 und 55:45, bezogen auf das Volumen, liegt.

10. Kosmetische Zusammensetzung, umfassend mindestens zu 0,1 Gew.-% einen Wirkstoff nach einem der Ansprüche 1 bis 9,
**dadurch gekennzeichnet, dass** sie in Form eines Shampoos, eines Haargels, einer Haarlotion, einer Haarmaske, eines Haar-Conditioners, einer Haarspülung oder einer Haarcreme auftritt.

11. Verfahren zum Erhalten eines Wirkstoffs nach einem der Ansprüche 1 bis 9, **dadurch gekennzeichnet, dass** es die folgenden Schritte umfasst:
a) Solubilisieren von Samenpulver von *Avena strigosa* in Wasser, gefolgt von enzymatischer Hydrolyse und enzymatischer Inaktivierung durch Wärmebehandlung,
b) Solubilisieren von Wurzelpulver von *Ononis spinosa* in einer Butylenglykol/Wasser-Mischung,
c) Mischen der aus den Schritten a) und b) entstandenen Produkte
d) Trennen der löslichen und der unlöslichen Phase,
e) Filtrieren.

12. Kosmetische Verwendung eines kosmetischen Wirkstoffs nach einem der Ansprüche 1 bis 9 auf den Haaren zum Vorbeugen und/oder Bekämpfen des Ergrauens von Haaren.

13. Kosmetische Verwendung einer kosmetischen Zusammensetzung nach Anspruch 10 auf den Haaren zum Vorbeugen und/oder Bekämpfen des Ergrauens von Haaren.

14. Kosmetische Verwendung nach einem der Ansprüche 12 oder 13 zum Erhalten der Pigmentierung von Haaren und/oder zum Verbessern der Strukturqualität von Haaren und/oder zum Reduzieren der Anzahl grauer Haare.

15. Verfahren zum kosmetischen Behandeln des Haars eines Menschen, zum Vorbeugen und/oder Bekämpfen des Ergrauens von Haaren,
**dadurch gekennzeichnet, dass** es darin besteht, mindestens einmal täglich eine Zusammensetzung nach Anspruch 10 auf die Haare aufzutragen.

## Claims

1. Cosmetic active ingredient comprising at least one extract of *Avena strigosa* and at least one extract of *Ononis spinosa.*

2. Cosmetic active ingredient according to claim 1,
**characterized in that** it comprises at least one extract of seeds of *Avena strigosa* and at least one extract of roots of *Ononis spinosa.*

3. Cosmetic active ingredient according to one of the preceding claims, **characterized in that** it comprises at least peptides.

4. Cosmetic active ingredient according to one of the preceding claims, **characterized in that** it comprises at least peptides with molar masses less than 2000 Da, said peptides represent at least 30 wt.% of dry matter of the active ingredient.

5. Cosmetic active ingredient according to one of the preceding claims, **characterized in that** it comprises at least phenolic compounds.

6. Cosmetic active ingredient according to one of the preceding claims, **characterized in that** it comprises at least phenolic acids and flavonoids, said phenolic acids and flavonoids represent at least 22 wt. % of dry matter of the active ingredient.

7. Cosmetic active ingredient according to one of the preceding claims, **characterized in that** the extract of *Avena strigosa* is a hydrolyzate of *Avena strigosa.*

8. Cosmetic active ingredient according to one of the preceding claims, **characterized in that** the extract of *Ononis spinosa* is a hydroglycolic extract of *Ononis spinosa.*

9. Cosmetic active ingredient according to one of the preceding claims, **characterized in that** the ratio of *Avena strigosa extract*/*Ononis spinosa* extract is between 45/55 and 55/45 by volume.

10. Cosmetic composition comprising at least 0.1 wt.% of an active ingredient according to one of claims 1 to 9, **characterized in that** it is provided in the form of shampoo, hair gel, hair lotion, hair mask, hair conditioner, conditioner, hair cream.

11. Method for obtaining an active ingredient according to one of claims 1 to 9, **characterized in that** it comprises the following steps:
a/ Solubilizing powdered seeds of *Avena strigosa* in water followed by enzymatic hydrolyses and enzymatic inactivation by heat treatment,
b/ Solubilizing powdered roots of *Ononis spinosa* in a Butylene Glycol/water mixture,
c/ Mixing products from steps a/ and b/,
d/ Separating the soluble and insoluble phases,
e/ Filtration.

12. Cosmetic use of a cosmetic active ingredient according to one of claims 1 to 9 on the hair, to prevent and/or combat graying of the hair.

13. Cosmetic use of a cosmetic composition according to claim 10 on the hair, for preventing and/or combating graying of the hair.

14. Cosmetic use according to one of claims 12 or 13, for preserving the pigmentation of the hair and/or for improving the structural quality of the hair and/or for reducing the number of gray hairs.

15. Method for the cosmetic treatment of the hair of a human being, for preventing and/or combating graying of the hair, **characterized in that** it consists in applying to the hair at least once a day a composition according to claim 10.
